# EUROPEAN PATENT APPLICATION

(11) **EP 4 821 509 A1**
(43) Date of publication of application: **14.10.2026**
(21) Application number: 25760655.8
(22) Date of filing: 14.01.2025
(51) Int. Cl.: A61K 45/06, A61K 31/517, A61K 31/506, A61P 35/00, A61P 35/04

(54) **USE OF QUINAZOLINE COMPOUND AND THIRD-GENERATION EGFR INHIBITOR IN COMBINED DRUG FOR TREATING NON-SMALL CELL LUNG CANCER**

(30) Priority: 26.02.2024 CN 202410209314
(71) Applicant: WEISHANG (HANGZHOU) BIO-PHARMACEUTICAL CO., LTD., Hangzhou, Zhejiang 310018 (CN)
(72) Inventor: ZHONG, Wei, Shanghai 201100 (CN)
(74) Representative: Huang, Liwei
(86) International application number: PCT/CN2025/072184
(87) International publication number: WO 2025/180111

(57) **Abstract**

The present disclosure provides use of a quinazoline compound and a third-generation EGFR (epidermal growth factor receptor) inhibitor in a combination medicament for treating non-small cell lung cancer, wherein the quinazoline compound comprises at least one selected from a quinazoline derivative represented by Formula I and salts, solvates, hydrates, and polymorphs thereof, 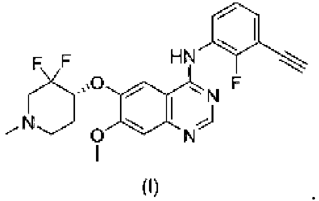 The composition of the quinazoline derivative (I) and a pharmaceutically acceptable salt thereof and the third-generation EGFR inhibitor of the present disclosure has an unexpected synergistic effect, and exhibits better efficacy than either single agent, for example, against non-small cell lung cancer driven by EGFR L858R or Del19 mutation, brain metastasis of non-small cell lung cancer, and leptomeningeal metastasis of non-small cell lung cancer; and the composition of the quinazoline derivative (I) and a pharmaceutically acceptable salt thereof and the third-generation EGFR inhibitor can prolong the time required for non-small cell lung cancer cells driven by EGFR L858R or Del19 mutation to develop drug resistance, and prolong the progression-free survival of the disease.

## Description

### TECHNICAL FIELD

The present disclosure relates to a use of a quinazoline compound and a third-generation EGFR inhibitor in a combination medicament for treating non-small cell lung cancer, belonging to the technical field of biomedicine.

### BACKGROUND ART

A quinazoline derivative capable of crossing the blood-brain barrier, with a molecular formula C₂₃H₂₁F₃N₄O₂ and the chemical name (R)-6-[(3,3-difluoro-1-methylpiperidin-4-yl)oxy]-N-(3-ethynyl-2-fluorophenyl)-7-methoxyquin azolin-4-amine (I), is a molecularly targeted anti-tumor drug, which is a highly selective epidermal growth factor receptor (EGFR) tyrosine kinase inhibitor, and can be used for treating brain metastasis of non-small cell lung cancer, leptomeningeal metastasis, head and neck squamous cell carcinoma, squamous cell carcinoma, brainstem tumor, primary brain cancer, brain glioma, or other cancers. As is known to those skilled in the art, a single agent having dose-dependent efficacy may effectively inhibit the growth of cancer cells at high doses, but may produce greater toxic side effects. Therefore, it is very useful and of great significance to develop a pharmaceutical composition capable of producing a synergistic effect to improve the efficacy of a single agent, minimize the toxic side effects of the second therapeutic agent or the compound (I) of the present disclosure, improve efficacy, improve the convenience of administration or use, and/or reduce the overall cost of the compound product or formulation.

### SUMMARY OF THE DISCLOSURE

In view of the deficiencies in the prior art, the object of the present disclosure is to provide a use of a quinazoline compound and a third-generation EGFR inhibitor in a combination medicament for treating non-small cell lung cancer.

The object of the present disclosure is achieved through the following technical solutions:

The present disclosure provides use of a quinazoline compound and a third-generation EGFR inhibitor in a combination medicament for treating non-small cell lung cancer. The quinazoline compound of the present disclosure has its unique properties, such as an ATP non-competitive binding mechanism with EGFR, potentially overcoming multiple drug resistance mechanisms of various third-generation inhibitors, so that the combined administration produces a synergistic effect, thereby producing improved therapeutic effects. Meanwhile, the dosage needs to reach a certain range (for humans, the dosage is 80-240 mg/day) to achieve improved effects; an excessively high dose may cause intolerable toxicity, and an excessively low dose may fail to achieve improved efficacy.

As one embodiment of the present disclosure, the quinazoline compound comprises at least one selected from a quinazoline derivative represented by Formula I and salts, solvates, hydrates, and polymorphs thereof,

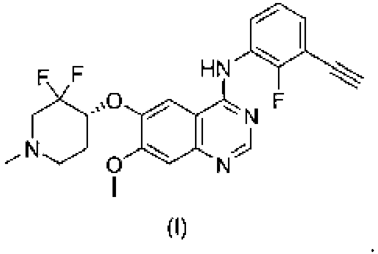

As one embodiment of the present disclosure, the quinazoline compound comprises at least one selected from a hydrochloride, sulfate, maleate, succinate, adipate, glycolate, malate, fumarate, benzenesulfonate, benzoate, hippurate, and oxalate, as well as solvates, hydrates, and polymorphs of the quinazoline derivative.

As one embodiment of the present disclosure, the non-small cell lung cancer comprises leptomeningeal metastasis of non-small cell lung cancer and brain metastasis of non-small cell lung cancer.

As one embodiment of the present disclosure, the third-generation EGFR inhibitor is in an oral dosage form.

As one embodiment of the present disclosure, the quinazoline compound (I) is in an oral dosage form.

As one embodiment of the present disclosure, the dosage ratio of the quinazoline compound (I) to the third-generation EGFR inhibitor is from 4:1 to 1:2.

As one embodiment of the present disclosure, the third-generation EGFR inhibitor comprises at least one selected from a pyrimidine derivative that irreversibly binds to EGFR cysteine 797 (C797) to form a covalent bond, and salts, solvates, hydrates, and polymorphs thereof.

As one embodiment of the present disclosure, the third-generation EGFR inhibitor is selected from Osimertinib (AZD9291, Tagrisso), Furmonertinib (Ivesa), Almonertinib (Ameile), Befotertinib (Surmana), Oritinib (SH-1028), Rezivertinib (BPI-7711), Limertinib (ASK120067), Lazertinib (YH25448, LECLAZA), or TY-9591.

Compared with the prior art, the present disclosure has the following beneficial effects:
1. The composition of the quinazoline derivative (I) and a pharmaceutically acceptable salt thereof and the third-generation EGFR inhibitor of the present disclosure has an unexpected synergistic effect, and exhibits better efficacy than either single agent, for example, against non-small cell lung cancer driven by EGFR L858R or Del19 mutation, brain metastasis of non-small cell lung cancer, and leptomeningeal metastasis of non-small cell lung cancer;
2. The composition of the quinazoline derivative (I) and a pharmaceutically acceptable salt thereof and the third-generation EGFR inhibitor of the present disclosure can prolong the time required for non-small cell lung cancer cells driven by EGFR L858R or EGFR Del19 mutation to develop drug resistance, and prolong the progression-free survival of the disease.

### BRIEF DESCRIPTION OF THE DRAWINGS

By reading the detailed description of the non-limiting embodiments with reference to the following drawings, other features, objects, and advantages of the present disclosure will become more apparent:
FIG. 1 shows the pharmacodynamic experiment of the combined administration in a subcutaneous mouse model of BaF3 EGFR Del19 cancer cells;
FIG. 2 shows the pharmacodynamic experiment of the combined administration in a subcutaneous mouse model of BaF3 EGFR L858R cancer cells.

### DETAILED DESCRIPTION

The present disclosure will be described in detail below in conjunction with specific examples. The following examples will help those skilled in the art to further understand the present disclosure, but do not limit the present disclosure in any form. It should be noted that, for those of ordinary skill in the art, several modifications and improvements can also be made without departing from the concept of the present disclosure. These all fall within the protection scope of the present disclosure.

### Example 1

Inhibitory effect of the combination of osimertinib (third-generation EGFR inhibitor)-the quinazoline derivative (I) of the present disclosure on the growth of non-small cell lung cancer BaF3 EGFR Del19 mouse xenograft tumor

Non-small cell lung cancer BaF3 EGFR Del19 cells were cultured in vitro in suspension, with the culture conditions of RPMI 1640 medium supplemented with 10% fetal bovine serum, 1% penicillin/streptomycin/amphotericin B, in a 37°C, 5% CO₂ incubator. Routine treatment and passage were performed twice a week. When the cell saturation reached 80%-90% and the quantity reached the requirement, the cells were collected and counted, and 0.1 mL of 0.5×10⁶ cells (with Matrigel added, at a volume ratio of 1:1) of BaF3 EGFR Del19 cells were subcutaneously inoculated in the right hind back of each NOD SCID mouse (female, 6-8 weeks old, weight 18-22 g). When the average tumor volume reached approximately 100-150 mm³, the pharmacodynamic experiment was initiated with grouping and dosing. The antitumor effect of the test drugs was dynamically observed by measuring tumor diameter. The tumor diameter was measured with a vernier caliper twice a week. The calculation formula of tumor volume is: V = 0.5a × b², where a and b represent the long diameter and short diameter of the tumor, respectively. The tumor inhibitory efficacy of the compounds was evaluated by TGI (%) or relative tumor proliferation rate T/C (%). TGI (%) reflects the tumor growth inhibition rate. Calculation of TGI (%): TGI(%) = [(1-(average tumor volume of a treatment group at the end of administration - average tumor volume of the treatment group at the beginning of administration))/(average tumor volume of the solvent control group at the end of treatment - average tumor volume of the solvent control group at the beginning of treatment)] × 100%; wherein the solvent is 0.5% HPMC with 0.1% tween-80 in water. Relative tumor proliferation rate T/C (%): the calculation formula is as follows: T/C % = T_{RTV} / C_{RTV} × 100% (T_{RTV}: RTV of the treatment group; C_{RTV}: RTV of the negative control group). The relative tumor volume (RTV) was calculated based on the tumor measurement results, and the calculation formula is RTV = Vₜ / V₀, where V₀ is the average tumor volume measured at the time of group dosing (i.e., d0), and Vₜ is the average tumor volume at a certain measurement; T_{RTV} and C_{RTV} take data from the same day. The tumor diameter was measured twice a week, and the mouse weight was also measured at the same time as each measurement. The grouping and dosing regimens were as follows:

The osimertinib group was administered by gavage at 5 mg/kg (5 mg/kg is the equivalent dose of 80 mg approved in humans for osimertinib. A lower dose has worse efficacy than 5 mg/kg, while a higher dose cannot be tolerated in humans due to excessive toxic side effects), once a day. The quinazoline derivative (I) group of the present disclosure was administered by gavage at a dose of 10 mg/kg, twice a day. In the osimertinib-quinazoline derivative (I) of the present disclosure combination administration group, osimertinib was administered by gavage at 5 mg/kg once a day, and the quinazoline derivative (I) of the present disclosure was administered by gavage at a dose of 10 mg/kg twice a day. The negative control group was administered by gavage with an aqueous solution of 0.5% hydroxypropyl methylcellulose and 0.1% tween 80 (0.5% HPMC with 0.1% tween-80), twice a day.

### Example 2

### Pharmacodynamics in a subcutaneous mouse model of BaF3 EGFR Del19 tumor cells

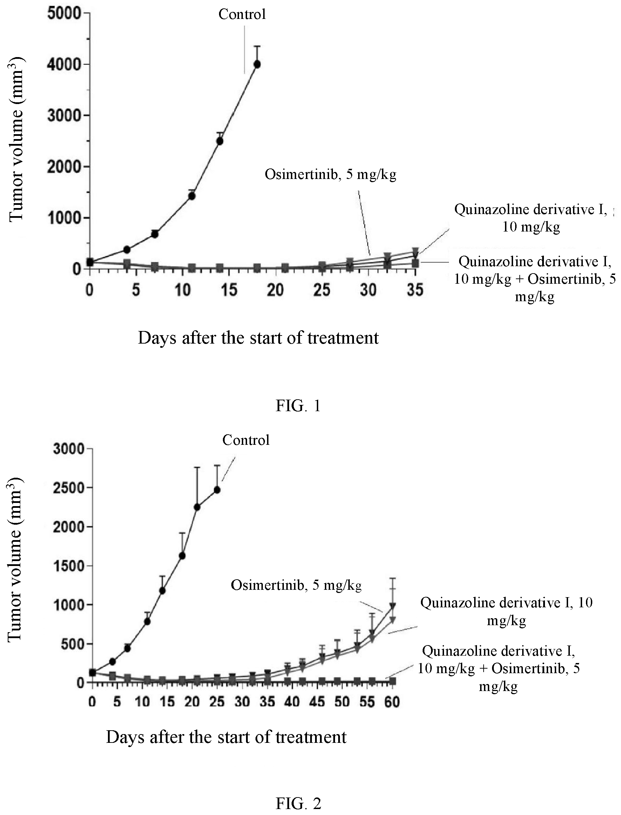

As shown in FIG. 1, in the pharmacodynamic experiment of subcutaneous mouse model of BaF3 EGFR Del19 tumor cells, administration was started on day 8 of tumor cell implantation. The second group, osimertinib (third-generation EGFR inhibitor), single agent, 5 mg/kg, oral, once a day, and the third group, quinazoline derivative (I), single agent, 10 mg/kg, oral, twice a day, both showed good inhibition of tumor growth compared with the first group control group (drug-free group) at 18 days after administration, showing good inhibition of tumor growth compared with the blank group, with statistically significant efficacy.

**TABLE 1**

| Group | Tumor volume (mm³) (day 18 after administration) | Relative tumor proliferation rate T/C (%) | TGI (%) | *p*-value (vs. control group) |
|---|---|---|---|---|
| Control group | 4003±351 | - | - | - |
| Osimertinib (third-generation EGFR inhibitor), 5 mg/kg, once a day | 24±7 | 0.6 | 102.8 | 0.0007 |
| Quinazoline derivative (I) of the present disclosure group, 10 mg/kg, twice a day | 24±7 | 0.6 | 102.8 | 0.0007 |
| Osimertinib (third-generation EGFR inhibitor), 5 mg/kg, once a day - Quinazoline derivative (I) of the present disclosure, 10 mg/kg, twice a day, combination administration group | 13±3 | 0.3 | 103.1 | 0.0007 |

The fourth group of the pharmaceutical composition of the present disclosure (quinazoline derivative (I) and osimertinib combination administration group) showed a synergistic effect on day 35 after administration, and improved the efficacy of the single agent compared with the second group and the third group single agent groups, with statistically significant efficacy differences, and prolonged the time required for cancer cells to develop drug resistance.

**TABLE 2**

| Group | Tumor volume (mm³) (day 35 after administration ) | *p*-value (vs. combination administration group) |
|---|---|---|
| Osimertinib (third-generation EGFR inhibitor), 5 mg/kg, once a day | 341±38 | 0.0021 |
| Quinazoline derivative (I) of the present disclosure group, 10 mg/kg, twice a day | 260±44 | 0.0329 |
| Osimertinib (third-generation EGFR inhibitor), 5 mg/kg, once a day - Quinazoline derivative (I) of the present disclosure, 10 mg/kg, twice a day, combination administration group | 111±12 | - |

### Example 3

Inhibitory effect of the combination of osimertinib (third-generation EGFR inhibitor) and the quinazoline derivative (I) of the present disclosure on the growth of non-small cell lung cancer BaF3 EGFR L858R mouse xenograft tumor

Non-small cell lung cancer BaF3 EGFR L858R cells were cultured in vitro in suspension, with the culture conditions of RPMI 1640 medium supplemented with 10% fetal bovine serum, 1% penicillin/streptomycin/amphotericin B, in a 37°C, 5% CO₂ incubator. Routine treatment and passage were performed twice a week. When the cell saturation reached 80%-90% and the quantity reached the requirement, the cells were collected and counted, and 0.2 mL of 1×10⁶ cells (with Matrigel added, at a volume ratio of 1:1) of BaF3 EGFR L858R cells were subcutaneously inoculated in the right hind back of each NOD SCID mouse (female, 6-8 weeks old, weight 18-22 g). When the average tumor volume reached approximately 100-150 mm³, the pharmacodynamic experiment was initiated with grouping and dosing. The antitumor effect of the test drugs was dynamically observed by measuring tumor diameter. The tumor diameter was measured with a vernier caliper twice a week. The calculation formula of tumor volume is: V = 0.5a × b², where a and b represent the long diameter and short diameter of the tumor, respectively. The tumor inhibitory efficacy of the compounds was evaluated by TGI (%) or relative tumor proliferation rate T/C (%). TGI (%) reflects the tumor growth inhibition rate. Calculation of TGI (%): TGI(%) = [(1-(average tumor volume of a treatment group at the end of administration - average tumor volume of the treatment group at the beginning of administration))/(average tumor volume of the solvent control group at the end of treatment - average tumor volume of the solvent control group at the beginning of treatment)] × 100%. Relative tumor proliferation rate T/C (%): the calculation formula is as follows: T/C % = T_{RTV} / C_{RTV} × 100% (T_{RTV}: RTV of the treatment group; C_{RTV}: RTV of the negative control group). The relative tumor volume (RTV) was calculated based on the tumor measurement results, and the calculation formula is RTV = Vₜ / V₀, where V₀ is the average tumor volume measured at the time of group dosing (i.e., d0), and Vₜ is the average tumor volume at a certain measurement; T_{RTV} and C_{RTV} take data from the same day. The tumor diameter was measured twice a week, and the mouse weight was also measured at the same time as each measurement. The grouping and dosing regimens were as follows:

The osimertinib group was administered by gavage at 5 mg/kg, once a day. The quinazoline derivative (I) group of the present disclosure was administered by gavage at a dose of 10 mg/kg, twice a day. In the osimertinib-quinazoline derivative (I) of the present disclosure combination administration group, osimertinib was administered by gavage at 5 mg/kg once a day, and the quinazoline derivative (I) of the present disclosure was administered by gavage at a dose of 10 mg/kg twice a day. The negative control group was administered by gavage with an aqueous solution of 0.5% hydroxypropyl methylcellulose and 0.1% tween 80 (0.5% HPMC with 0.1% tween-80), twice a day.

### Example 4

### Pharmacodynamics in a subcutaneous mouse model of BaF3 EGFR L858R tumor cells

As shown in FIG. 2, in the pharmacodynamic experiment of the subcutaneous mouse model of BaF3 EGFR L858R tumor cells, administration was started on day 8 of tumor cell implantation. The second group, quinazoline derivative (I), single agent, 10 mg/kg, oral, twice a day, and the third group, osimertinib (third-generation EGFR inhibitor), single agent, 5 mg/kg, oral, once a day, both showed good inhibition of tumor growth compared with the first group control group (drug-free group), with statistically significant efficacy.

**TABLE 3**

| Group | Tumor volume (mm³) (day 21 after administration) | T/C (%) | TGI (%) | *p*-value (vs. control group) |
|---|---|---|---|---|
| Control group | 2,252±511 | - | - | - |
| Osimertinib (third-generation EGFR inhibitor), 5 mg/kg, once a day | 28±8 | 1.3 | 104.8 | <0.0001 |
| Quinazoline derivative (I) of the present disclosure group, 10 mg/kg, twice a day | 46±13 | 2.1 | 104.0 | <0.0001 |
| Osimertinib (third-generation EGFR inhibitor), 5 mg/kg, once a day - Quinazoline derivative (I) of the present disclosure, 10 mg/kg, twice a day, combination administration group | 18±5 | 0.8 | 105.3 | <0.0001 |

The fourth group of the pharmaceutical composition of the present disclosure (quinazoline derivative (I) and osimertinib combination administration group) showed a synergistic effect on day 60 after administration, and improved the efficacy of the single agent compared with the second group and the third group single agent groups, with statistically significant efficacy differences, and prolonged the time required for cancer cells to develop drug resistance.

**TABLE 4**

| Group | Tumor volume (mm³) (day 60 after administration) | *p-value* (vs. combination administration group) |
|---|---|---|
| Osimertinib (third-generation EGFR inhibitor), 5 mg/kg, once a day | 800±405 | 0.0002 |
| Quinazoline derivative (I) of the present disclosure group, 10 mg/kg, twice a day | 974±365 | 0.0282 |
| Osimertinib (third-generation EGFR inhibitor), 5 mg/kg, once a day - Quinazoline derivative (I) of the present disclosure, 10 mg/kg, twice a day, combined administration group | 22±7 | - |

The specific examples of the present disclosure have been described above. It should be understood that the present disclosure is not limited to the specific embodiments described above, and those skilled in the art can make various modifications or alterations within the scope of the claims, which do not affect the substantive content of the present disclosure.

## Claims

1. A quinazoline compound in combination with a third-generation EGFR inhibitor for use in treating non-small cell lung cancer.

2. Product for use according to claim 1, wherein the quinazoline compound comprises at least one selected from a quinazoline derivative represented by Formula I, and salts, solvates, hydrates, and polymorphs thereof,

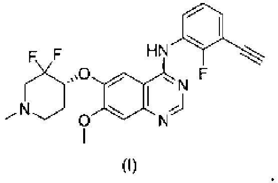

3. Product for use according to claim 1 or 2, wherein the quinazoline compound comprises at least one selected from a hydrochloride, sulfate, maleate, succinate, adipate, glycolate, malate, fumarate, benzenesulfonate, benzoate, hippurate, oxalate, solvate, hydrate, and polymorph of the quinazoline derivative.

4. Product for use according to claim 1, wherein the third-generation EGFR inhibitor comprises at least one selected from pyrimidine derivatives that irreversibly bind to EGFR cysteine 797 to form a covalent bond, and salts, solvates, hydrates, and polymorphs thereof.

5. Product for use according to claim 1 or 4, wherein the third-generation EGFR inhibitor is selected from osimertinib, furmonertinib, almonertinib, befotertinib, oritinib, rezivertinib, limertinib, lazertinib, or TY-9591.

6. Product for use according to claim 1, wherein the third-generation EGFR inhibitor is administrated in an oral dosage form.

7. Product for use according to claim 1, wherein the quinazoline compound is administrated in an oral dosage form.

8. Product for use according to claim 1, wherein the dose ratio of the quinazoline compound to the third-generation EGFR inhibitor is from 4:1 to 1:2.

9. Product for use according to claim 1, wherein the non-small cell lung cancer includes leptomeningeal metastasis of non-small cell lung cancer and brain metastasis of non-small cell lung cancer.
